# EUROPEAN PATENT SPECIFICATION

(11) **EP 2 326 957 B1**
(45) Date of publication and mention of the grant of the patent: **12.06.2013**
(21) Application number: 09792157.1
(22) Date of filing: 02.09.2009
(51) Int. Cl.: G01N 33/569

(54) **ASSAYS AND KITS FOR DETERMINING HIV-1 TROPISM**
TESTS UND KITS ZUR BESTIMMUNG VON HIV-1-TROPISMUS
DOSAGES ET KITS POUR DÉTERMINER UN TROPISME DU VIH-1

(30) Priority: 03.09.2008 US 94009 P
(43) Date of publication of application: 01.06.2011
(73) Proprietor: Abbott Molecular Inc., Des Plaines, IL 60018 (US)
(72) Inventor: LAFFLER, Thomas, G., Libertyville, IL 60048 (US)
(74) Representative: Modiano, Micaela Nadia
(86) International application number: PCT/US2009/055690
(87) International publication number: WO 2010/028014

(56) References cited:
- NAVRATILOVA ET AL: "Analyzing ligand and small molecule binding activity of solubilized GPCRs using biosensor technology" ANALYTICAL BIOCHEMISTRY, ACADEMIC PRESS INC, NEW YORK, vol. 355, no. 1, 1 August 2006 (2006-08-01), pages 132-139, XP005542212 ISSN: 0003-2697
- POVEDA EVA ET AL: "HIV tropism: diagnostic tools and implications for disease progression and treatment with entry inhibitors" AIDS (HAGERSTOWN), vol. 20, no. 10, June 2006 (2006-06), pages 1359-1367, XP002566591 ISSN: 0269-9370
- CANTIN REJEAN ET AL: "A novel virus capture assay reveals a differential acquisition of host HLA-DR by clinical isolates of human immunodeficiency virus type 1 expanded in primary human cells depending on the nature of producing cells and the donor source" JOURNAL OF GENERAL VIROLOGY, vol. 82, no. 12, December 2001 (2001-12), pages 2979-2987, XP002566592 ISSN: 0022-1317

## Description

### TECHNICAL FIELD

The present invention relates to assays and kits for determining the tropism of an human immunodeficiency virus-1 ("HIV-1") population in a test sample obtained from a subject.

### BACKGROUND

Human immunodeficiency virus ("HIV") is a human retrovirus that is an etiologic agent of acquired immune deficiency syndrome ("AIDS"), an infectious disease characterized by a profound loss of immune system function. Two types of HIV are known, HIV-1 and HIV-2.

The globally circulating strains of HIV-1 exhibit extreme genetic diversity (See, Robertson et al., Nature. 374:124 (1995)). To evaluate the extent of global HIV-1 variation, sequences of virus strains originating from numerous countries have been compared. These studies have shown that HIV-1 can be classified into two major groups, namely, Group M (for "Major") and Group O (for "Outlier"). HIV-1 Group M and Group O are distinct clusters on phylogenetic trees. Group M comprises the great majority of HIV-1 isolates and can be further subdivided into at least nine sequence subtypes or clades, designated A to 1, with additional variants being added continually to the classification scheme (*See*, Gao et al., J. Virol., 70:1651 (1996)). Although Group O strains are highly divergent, the clinical course of Group O infection is identical to that of HIV-1 Group M infection.

The main cells targeted by HIV-1 are T-cells, macrophages and dendritic cells *(See,* Clapham P. et al., British Medical Bulletin, 58:43-49 (2001)). HIV-1 interacts with several receptors on the surfaces of these cells to trigger the fusion of viral and cellular membranes in order to confer virus entry into these cells. *Id.* HIV-1 tropism refers to the types of cells that HIV-1 infects and the means by which infection is accomplished.

Specifically, HIV-1 interacts with the CD4 glycoprotein and a seven transmembrane (7TM) co-receptor to trigger entry into cells. *Id*. More specifically, HIV-1 contains envelope glycoprotein "spikes" on its surface that comprise an outer surface protein called gp120 which is non-covalently linked to a transmembrane protein, gp41. Each "spike" on an HIV-1 particle comprises a trimer of three gp120 proteins and three gp41 proteins. *Id*. The binding of CD4 to gp120 triggers a structural change that exposes a binding site for a co-receptor. *Id*. Further structural rearrangements are initiated when the co-receptor is bound. These changes are believed to be sufficient to trigger the fusion of viral and cellular membranes thus allowing entry of the virion core into the cytoplasm of a cell. *Id*.

Over fourteen different 7TM receptors have been identified as potential co-receptors for HIV-1. These receptors are either members of, or closely related to, the chemokine receptor family. Two major co-receptors of the chemokine receptor family are CCR5 and CXCR4. All HIV-1 isolates use one or both of these co-receptors. Specifically, the CCR5 receptor is the major co-receptor for macrophage-tropic (R5) strains, which play a crucial role in the sexual transmission of HIV-1. T cell line-tropic (X4) viruses use CXCR4 to enter target cells. CXCR4-using viruses tend to emerge in the later stages of infection in around 60% of progressing patients and their emergence coincides with accelerated disease progression (*See*, Westby M., et al.. J. of Virol., 80(10):4909-4920 (2006)). However, whether CXCR4 emergence is a cause or a consequence of severe immune system impairment is unknown, as little is known about the mechanism by which CXCR4 viruses are selected during the course of infection. *Id*. Some HIV-1 isolates are dualtropic (R5X4) since they can use both co-receptors, although not always with the same efficiency.

The CCR5 and CXCR4 co-receptors are attractive targets for drug development since they are members of the G protein-coupled receptor superfamily, a group of proteins targeted by several commonly used and well-tolerated drugs, such as destoradine, ranitidine and tegaserod. *Id*. CCR5 is of particular interest since a natural polymorphism exists in humans (CCR5-Δ32) that leads to reduced or absent cell surface expression of CCR5 in heterozygotic or homozygotic genotypes, respectively. *Id*. Individuals homozygotic for CCR5-Δ32 appear to benefit from a natural resistance to HIV-1 infection, while heterozygotic CCR5-Δ32 is associated with reduced disease progression. *Id*. Antagonists that block the binding of HIV-1 to the CCR5 are being developed as the first anti-HIV agents acting on a host target cell. However, these antagonists are only effective in patients lacking the CXCR4 co-receptor. Currently, there is a belief that selective inhibition of CCR5-using strains by treatment with CCR5 antagonists may lead to an increased rate of emergence of CXCR4 variants. *Id*.

A number of assays for determining the tropism of an HIV-1 population are known in the art. These assays involve either determining the binding to receptors displayed on cell surfaces or inferring tropism from genetic information. For example, U.S. Patent No. 7,294,458 describes an assay that involves transforming cells with an HIV envelope gene cloned from an infected patient, selectively fusing the cells with an indicator cell line that expresses an HIV envelope-compatible co-receptor and then assaying for fusion. Cell surface envelope protein variants selectively interact with either CCR5 or CXCR4 co-receptors. Fusion occurs only when an envelope protein interacts with a compatible co-receptor present on the surface of indicator cells. Cells expressing a particular envelope gene will fuse either CCR5 or CXCR4 indicator cells depending on the patient's envelope gene specificity. Fusion with either CCR5 or CXCR4 indicator cells indicates the type of co-receptor usage.

As illustrated by the astay described in U.S. Patent No. 7,294,458, the HIV-1 tropism assays known in the art are time consuming (namely, they take longer than 24 hours to provide a result) and are expensive. Therefore, there is a need in the art for assays for determining the tropism of an HIV-1 population that can be performed rapidly, namely in 24 hours or less and are less expensive to perform than assays currently known in the art.

The Article of Navrativola et al. (Analytical Biochemistry, Academic Press Inc, New York, vol. 335, no.1, (2006-08-01), pages 132-139) refers to an analysis of ligand and small molecule binding activity of solubilized GPCRs using biosensor technology, and discloses CXCR4 or CCR5 immobilized on a solid phase.

The review of Poveda et al. (AIDS (HAGERSTOWN), vol. 20, no.10, (2006-06), pages 1359-1367) refers to methods for determining HIV tropism.

The article of Cantine et al. (Journal of General Virology, vol. 82, no. 12 (2001-12), pages 2979-2987) discloses an affinity capture assay of HIV virions in the context of viral tropism using anti-HLA antibodies as binding partner.

### SUMMARY

In one embodiment, the present invention relates to an assay for detecting changes in tropism of an HIV-1 population for a chemokine receptor in a test sample obtained from a subject. The assay comprises the steps of:
(a) contacting the test sample obtained from the subject with a first specific binding partner and a second specific binding partner to form a first specific binding partner-NIV-1 complex and a second specific binding partner-HIV-1 complex, wherein the first specific binding partner comprises a CCR5 co-receptor protein and the second specific binding partner comprises a CXCR4 co-receptor protein;
(b) separating the first specific binding partner-HIV-1 complex and second specific binding partner HIV-1 complex from the test sample;
(c) isolating RNA from the first specific binding partner-HIV-1 complex and RNA from the second specific binding partner-HIV-1 complex;
(d) quantifying the isolated RNA from the first specific binding partner-HIV-1 complex and the isolated RNA from the second specific binding partner-HIV-1 complex and determining a ratio of HIV-1 virus binding to CCR5 co-receptor protein to HIV-1 virus binding to CXCR4 co-receptor protein; and
(e) comparing the ratio determined in step (d) with a predetermined ratio of HIV-1 virus binding to a CCR5 co-receptor protein to HIV-1 virus binding to a CXCR4 co-receptor protein, wherein a change in the ratio compared to the predetermined ratio indicates a change in tropism in the HIV-1 population in the subject.

In the above assay, the first specific binding partner, the second specific binding partner or both the first specific binding partner and the second specific binding partner can be immobilized on a solid phase either before or after contact with the test sample. The solid phase is selected from the group consisting of a magnetic particle, a non-magnetic particle, a membrane, a ligand, a receptor, a microparticle and a bead.

In the above assay, the isolated RNA from the first specific binding partner-HIV-1 complex and the isolated RNA from the second specific binding partner-HIV-1 complex are quantified using PCR, nucleic acid sequence based amplification, tissue microarrays, transcription mediated amplification or Northern Blot analysis.

In another embodiment, the present invention relates to an assay for monitoring whether a subject infected with an HIV-1 population has become tropic for a CXCR4 co-receptor. The assay comprises the steps of:
(a) contacting a test sample obtained from the subject with a first specific binding partner and a second specific binding partner to form a first specific binding partner-HIV complex and a second specific binding partner-HIV-1 complex, wherein the first specific binding partner comprises a CCR5 co-receptor protein and the second specific binding partner comprises a CXCR4 co-receptor protein further wherein said subject was previously identified as HIV-1 tropic for the CCR5 co-receptor;
(b) separating the first specific binding partner-HIV-1 complex and second specific binding partner HIV-1 complex from the test sample;
(c) isolating RNA from the first specific binding partner-HIV-1 complex and RNA from the second specific binding partner-HIV-1 complex;
(d) quantifying the isolated RNA from the first specific binding partner-HIV-1 complex and the isolated RNA from the second specific binding partner-HIV-1 complex, and determining a ratio of HIV-1 virus binding to CCR5 co-receptor protein to HIV-1 virus binding to CXCR4 co-receptor protein; and
(e) comparing the ratio determined in step (d) with a predetermined ratio of HIV-1 virus binding to a CCR5 co-receptor protein to HIV-1 virus binding to a CXCR4 co-receptor protein, wherein an increase in the ratio of CXCR4 co-receptor protein or CXCR4 analog thereof compared to the predetermined ratio indicates a change in tropism of the HIV-1 population from the CCR5 co-receptor protein to the CXCR4 co-receptor protein in the subject.

In the above assay, the first specific binding partner, the second specific binding partner or both the first specific binding partner and the second specific binding partner can be immobilized on a solid phase either before or after contact with the test sample. The solid phase is selected from the group consisting of a magnetic particle, a non-magnetic particle, a membrane, a ligand, a receptor, a microparticle and a bead.

In the above assay, the isolated RNA from the first specific binding partner-HIV-1 complex and the isolated RNA from the second specific binding partner-HIV-1 complex are quantified using PCR, nucleic acid sequence based amplification, tissue microarrays, transcription mediated amplification or Northern Blot analysis.

In the above assay, the subject may have been receiving treatment for the HIV-1 with one or more pharmaceutical compositions. An example of one or more pharmaceutical compositions is a CCR5 antagonist.

In yet another embodiment, the present invention relates to an assay for monitoring whether an HIV-1 population has become tropic for a CXCR4 co-receptor. The assay comprises the steps of:
(a) contacting a test sample obtained from a subject with a specific binding partner to form a first specific binding partner-HIV-1 complex, wherein the specific binding partner comprises a CXCR4 co-receptor protein or a CXCR4 analog thereof, and further wherein the specific binding partner is immobilized on a solid phase;
(b) separating the specific binding partner-HIV-1 complex from the test sample;
(c) isolating RNA from the specific binding partner-HIV-1 complex;
(d) quantifying the isolated RNA from the specific binding partner-HIV-1 complex and total HIV-1 RNA in the test sample and determining a ratio of RNA virus binding CXCR4 co-receptor protein to total HIV-1 RNA;
(e) comparing the ratio determined in step (d) with a predetermined ratio of HIV-1 virus binding to a CXCR4 co-receptor protein to total HIV-1 RNA, wherein an increase in the ratio of step (d) compared to the predetermined ratio indicates that the HIV-1 population has become tropic for the CXCR4 co-receptor protein.

The solid phase used in the above assay is selected from the group consisting of a magnetic particle, a non-magnetic particle, a membrane, a ligand, a receptor, a microparticle and a bead.

In the above assay, the isolated RNA from the specific binding partner-HIV-1 complex is quantified using PCR, nucleic acid sequence based amplification, tissue microarrays, transcription mediated amplification or Northern Blot analysis.

In the above assay, the subject may have been receiving treatment for the HIV-1 with one or more pharmaceutical compositions. An example of one or more pharmaceutical compositions is a CCR5 antagonist.

In still yet another embodiment, the present invention relates to an assay for monitoring whether an HIV-1 population has become tropic for a CXCR4 co-receptor. The assay comprises the steps of:
(a) contacting a test sample obtained from a subject with a specific binding partner to form a first specific binding partner-HIV complex, wherein the specific binding partner comprises a CXCR4 co-receptor protein, and further wherein the specific binding partner is immobilized on a solid phase;
(b) separating the specific binding partner-HIV-1 complex from the test sample;
(c) isolating RNA from the specific binding partner-HIV-1 complex;
(d) quantifying the isolated RNA from the specific binding partner-HIV-1 complex;
   and
(e) comparing the quantity of RNA from the specific binding partner-HIV-1 complex with a predetermined level of RNA binding to CXCR4 co-receptor protein wherein if the isolated RNA quantified from the specific binding partner-HIV-1 complex is less than the predetermined level, then the HIV-1 population has not become tropic for the CXCR4 co-receptor protein, and further wherein if the isolated RNA quantified from the specific binding partner-HIV-1 complex is greater than the predetermined level, then the HIV-1 population has become tropic for the CXCR4 co-receptor protein.

The solid phase used in the above assay is selected from the group consisting of a magnetic particle, a non-magnetic panicle, a membrane, a ligand, a receptor, a microparticle and a bead.

In the above assay, the isolated RNA from the specific binding partner-HIV-1 complex is quantified using PCR, nucleic acid sequence based amplification, tissue microarrays, transcription mediated amplification or Northern Blot analysis.

In the above assay, the subject may have been receiving treatment for the HIV-1 with one or more pharmaceutical compositions. Examples of one or more pharmaceutical compositions are CCR5 antagonist.

Examples of the types of PCR that can be used in the above assays are reverse transcriptase PCR (RT-PCR), competitive RT-PCR, real time RT-PCR or differential display RT-PCR.

In yet still another embodiment, the present invention relates to a kit for determining tropism of an HIV-1 population in a test sample obtained from a subject. Such a kit comprises:
(a) a first specific binding partner comprising a CXCR4 co-receptor;
(b) a second specific binding partner comprising a CCR5 co-receptor;
(c) at least one solid phase;
(d) one or more amplification reagents selected from the group consisting of one or more primer sets specific for a sequence of interest, a heat activated thermostable Taq polymerase, AMV reverse transcriptase, RNAaseH, T7RNA polymerase, RNA polymerase, a reverse transcriptase, deoxyribonucleotide triphoshates, MgCl₂, one or more detector probes, and one or more buffers, wherein the amplification reagents allow the user to perform one or more amplification reactions in order to quantify the amount of HIV RNA obtained from the test sample using any one of various types of transcripted mediated amplification (TMR), nucleic acid sequence based amplification (NASBA), polymerase chain reaction (PCR) selected from the group consisting of RT-PCR, competitive RT-PCR, real time RT-PCR, differential display RT-PCR; and
(e) instructions for using said kit;
wherein the amount of RNA from an HIV-1 virus binding to (a) and the amount of RNA from an HIV-1 virus binding to (b) are measured.

In still yet another embodiment, the present invention relates to another type of kit for determining tropism of an HIV-1 population in a test sample obtained from a subject. The kit can comprise:
(a) a first specific binding partner comprising a CCR5 co-receptor;
(b) a second specific binding partner comprising a CXCR4 co-receptor
(c) at least one solid phase;
(d) one or more amplification reagents selected from the group consisting of one or more primer sets specific for a sequence of interest, a heat activated thermostable Taq polymerase, AMV reverse transcriptase, RNAaseH, T7RNA polymerase, RNA polymerase, a reverse transcriptase, deoxyribonucleotide triphoshates, MgCl₂, one or more detector probes, and one or more buffers, wherein the amplification reagents allow the user to perform one or more amplification reactions in order to quantify the amount of HIV RNA obtained from the test sample using any one of various types of transcripted mediated amplification (TMR), nucleic acid sequence based amplification (NASBA), polymerase chain reaction (PCR) selected from the group consisting of RT-PCR, competitive RT-PCR, real time RT-PCR, differential display RT-PCR; and
(e) instructions for using said kit;
wherein the amount of RNA from an HIV-1 virus binding to (a) and the amount of RNA from an HIV-1 virus binding to (b) are measured.

### DETAILED DESCRIPTION

The present invention relates to assays and kits for determining the tropism of a human immunodeficiency virus ("HIV-1") population in test samples obtained from one or more subjects. The assays of the present invention can be performed relatively quickly, namely, in 24 hours or less. Also, because the assays of the present invention do not require the use of culturing techniques to provide a result, the assays of the present invention are less expensive than other assays known in the art.

Determining the tropism of an HIV-1 using the assays and kits of the present invention provides a clinician with useful information in determining the course of treatment in a subject as well as monitoring the course of treatment in a subject. For example, for a subject newly or recently diagnosed with HIV-1, the assays and kits of the present invention allow the clinician to determine whether or not the HIV-1 population in the test sample obtained from a subject is tropic for the CCR5 or CXCR4 co-receptor and thus prescribe the appropriate course of treatment for that subject. Alternatively, for a subject previously diagnosed with HIV-1 and receiving treatment with one or more pharmaceutical compositions, the assays and kits of the present invention allow the clinician to monitor whether or not during a course of treatment an HIV-1 population has become tropic for a different chemokine co-receptor.

### A. Definitions

As used herein, the singular forms "a," "an" and "the" include plural referents unless the context clearly dictates otherwise. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

### a) CCR5

The terms "CCR5", "CCR5 co-receptor" and "CCR5 co-receptor protein", are used interchangeably herein. They refer to a chemokine receptor which binds to members of the C-C group of chemokines. The CCR5, CCR5 co-receptor and CCR5 co-receptor protein can be human or non-human. The amino acid sequences of human and various non-human primate CCR5, CCR5 co-receptors and CCR5 co-receptor proteins are known in the art. Examples of CCR5, CCR5 co-receptors and CCR5 co-receptor proteins that can be used in the present invention include, but are not limited to, those shown in Table A.

**Table A**

| Species | GenBank Accession No. or other Source |
|---|---|
| *Homo supiens* | X91492 and 1705896 |
| *Pan troglodytes* | AF005663 |
| *Gorilla gorilla* | AF005659 |
| *Papio hamadryas* | AF005658 |
| *Papio hamadryas Anubis* | AF023452 |
| *Cercocebus torquatus atys* | AF079472 |
| *Macaca fascicu*/*aris* | AF005660 |
| *Macaca nemestrina* | AF005661 |
| *Cercopithecus aethiops 1* | U83324 |
| *Cercopithecus aethiops 2* | U83325 |
| *Pongo pygmaeus* | AF075446 |
| *Hylobates leucogenys* | AF075451 |
| *Trachypithecus francoisi* | AF075442 |
| *Trachypithecus phayrei* | AF075443 |
| *Pygathrix nemaeus* | AF075448 |
| *Rhinopithecus roxellanae* | AF075444 |
| *Rhinopithecus bieti* | AF075445 |
| *Rhinopithecus avunculus* | AF075447 |
| *Macaca assamensis* | AF075449 |
| *Macaca arctoides* | AF075450 |
| *Mus musculus* | AF022990 |

### b) CCR5 Antagonist

As used herein, the phrase "CCR5 antagonist" is a pharmaceutical composition that that can be used to block the binding of HIV-1 to the CCRS co-receptor. An example of a CCR5 antagonist is maravioc (See, Dorr, P., et al., Antmicrobi. Agents Chemother., 49:4721-4732 (2005)). Other examples utilize the antibodies described in U.S. Patent Publication US 2008/0107595.

### c) Chemokines

As used herein, the term "chemokines" refers to a cytokine that can stimulate leukocyte movement. Chemokines can be characterized either as CYS-CYS (hereinafter "C-C") or CYS-X-CYS (hereinafter "C-X-C") depending on whether the two amino terminal cysteine residues are immediately adjacent or separated by one amino acid. Examples include, but are not limited to, RANTES, MIP-1α, M1P-10, SDF-1 and any other chemokine which blocks HIV-1 infection.

### d) Chemokine Receptor

As used herein, the phrase "chemokine receptor" refers to a member of a homologous family of seven-transmembrane spanning cell surface proteins that bind chemokines. Preferred chemokine receptors are those that function as HIV-1 co-receptors, including, but not limited to, CCR2, CCR3, CCR5, CCR8, CXCR4, STRL33, GPR-15, CX3GR1 and APJ.

### e) CXCR4

The terms "CXCR4", "CXCR4 co-receptor" and "CXCR4 co-receptor protein" are used interchangeably herein. They refer to chemokine receptors that bind to members of the C-X-C group of chemokines. The CXCR4, CXCR4 co-receptor and CXCR4 co-receptor protein can be human or non-human primate. The amino acid sequences of human and various non-human primate CXCR4, CXCR4 co-receptors and CXCR4 co-receptor protein are known in the art. Examples of CXCR4, CXCR4 co-receptors or CXCR4 co-receptor proteins that can be used in the present invention include, but are not limited to, those shown in Table B.

**Table B**

| Species | GenBank Accession No. or other Source |
|---|---|
| *Homo sapiens* | 400654 and 85740 |
| *Callithrix jacchus* | AF452612 |
| *Saimiri sciureus* | AF452613 |

### f) CXCR4 Antagonist

As used herein, the phrase "CXCR4 antagonist" is a pharmaceutical composition that that can be used to block the binding of HIV-1 to the CXCR4 co-receptor. An example of a CXCR4 antagonist is 1,1'-[1,4-phenylenebis(methylene)]-bis-1,4,8, 11-tetraazacyclotetradecane octahydrochloride dihydrate (AMD3100) *(See,* De Clercq, E., Molecular Pharmacology, 57:833-839 (2000))

### g) Analogs of CCRS

The phrases "analogs of CCRS", "analogs of a CCR5 co-receptor protein", and "CCR5 analogs" are used interchangeably herein. They refer to binding analogs, including, but not limited to, antibodies, proteins, peptides, aptamers, salts, esters, amides, prodrugs, conjugates; active metabolites, and other such derivatives, analogs, polymorphic variants and related compounds of CCR5.

### h) Analogs of CXCR4

The phrases "analogs of CXCR4", "analogs of a CXCR4 co-receptor protein", and "CXCR4 analogs" are used interchangeably herein. They refer to binding analogs, including, but not limited to, antibodies, proteins, peptides, aptamers, salts, esters, amides, prodrugs, conjugates, active metabolites, and other such derivatives, analogs, polymorphic variants and related compounds of CXCR4.

### i) Pharmaceutical Composition

As used herein, the term "pharmaceutical composition" refers to any agent or drug, whether a small molecule (e.g., a drug containing an active agent, typically a non-peptidic) or biologic (e.g., a peptide, protein or antibody based drug, including any with modifications, such as, but not limited to PEGylation) that can be used to treat a subject suffering from HIV-1. Examples of pharmaceutical compositions, include, but are not limited to, nucleoside analogue reverse transcriptase inhibitors, protease inhibitors, non-nucleoside reverse transcriptase inhibitors, CCR5 antagonists, CXCR4 antagonists and the like.

### j) Predetermined Level or Predetermined Ratio

As used herein, the phrases "predetermined level" or "predetermined ratio" refer generally to an assay cutoff value that is used to assess diagnostic results by comparing the assay results against the predetermined level or predetermined ratio and where the predetermined level or predetermined ratio already has been linked or associated with various clinical parameters (e.g., assessing risk, detecting changes in tropism of a disease or virus, (e.g., whether an HIV-1 population has become tropic for a CXCR4 co-receptor), severity of disease, progression/nonprogression/improvement and the like)). A predetermined level or predetermined ratio can also be an individual reference value that has been determined from prior samples obtained from a specific subject. It is well known that cutoff values may vary dependent on the nature of the assay.

### k) Specific Binding Partner

As used herein, the phrase "specific binding partner" is a member of a specific binding pair (i.e., two different molecules where one of the molecules specifically binds to the second molecule through chemical or physical means. Therefore, in addition to antigen and antibody specific binding pairs of common immunoassays, other specific binding pairs can include biotin and avidin (or streptavidin), carbohydrates and lectins, complementary nucleotide sequences, effector and receptor molecules, cofactors and enzymes, enzyme inhibitors, and enzymes and the like. Furthermore, specific binding pairs can include members that are analogs of the original specific binding members, for-example, an analyte-analog. Immunoreactive specific binding members include antigens, antigen fragments, antibodies and antibody fragments, both monoclonal and polyclonal and complexes thereof, including those formed by recombinant DNA molecules.

### 1) Test Sample

As used herein, the term "test sample" generally refers to a biological material being tested for and/or suspected of containing an analyte of interest, such as HIV-1. The test sample may be derived from any biological source, such as, a physiological fluid, including, but not limited to, whole blood, serum, plasma, interstitial fluid, saliva, ocular lens fluid, cerebral spinal fluid, sweat, urine, milk, ascites fluid, mucous, nasal fluid, sputum, synovial fluid, peritoneal fluid, vaginal fluid, menses, amniotic fluid, semen and so forth. The test sample may be used directly as obtained from the biological source or following a pretreatment to modify the character of the sample. For example, such pretreatment may include preparing plasma from blood, diluting viscous fluids and so forth. Methods of pretreatment may also involve filtration, precipitation, dilution, distillation, mixing, concentration, inactivation of interfering components, the addition of reagents, lysing, etc.
Moreover, it may also be beneficial to modify a solid test sample to form a liquid medium or to release the analyte.

### B. Assays for Determining Tropism of a HIV-1 for a Chemokine Receptor in a Test Sample

The present invention relates to assays or methods for determining the tropism of an HIV-1 population for chemokine receptors in a test sample, determining changes in tropism in an HIV-1 population in a test sample and monitoring whether a subject infected with an HIV-1 population has become tropic for a chemokine co-receptor. The preferred chemokine receptors are the CCR5 co-receptor and the CXCR4 co-receptor.
The assays or methods of the present invention are practised on a test sample obtained from a subject. Subjects can be any vertebrate, and preferably, are mammals. Examples of mammals include, but are not limited to, dogs, cats, rabbits, mice, rats, goats, sheep, cows, pigs, horses, non-human primates and humans. The test sample can be obtained from the subject using routine techniques known to those skilled in the art. Preferably, the test sample contains HIV-1. Specifically, the test sample can be obtained from a subject that is suspected or believed to be infected with or suffering from HIV-1 or a subject already known to be suffering from HIV-1 and who may be optionally receiving treatment for HIV-1 with one or more pharmaceutical compositions.

A first mixture is then prepared. In one embodiment of the present invention, the first mixture contains the test sample, a first specific binding partner that comprises a first chemokine receptor and a second specific binding partner that comprises a second chemokine receptor. The order in which the test sample, the first specific binding partner and the second specific binding partner are added to form the first mixture is not critical. The first specific binding partner can comprise a CCR5 receptor (or CXCR4 receptor) as the first chemokine receptor and the second specific binding partner can comprise a CXCR4 receptor (or CCR5 receptor) as the second chemokine receptor. Essentially, it does not matter whether the first or second specific binding partner comprise the CCR5 receptor or CXCR4 receptor. What matters is that the first or second specific binding partners comprise chemokine receptors that are not identical.

If the first specific binding partner comprises a CCR5 receptor, the first specific binding partner and any HIV-1 in the test sample tropic for the CCR5 receptor will bind and form a first specific binding partner-HIV-1 complex. If the second specific binding partner comprises a CXCR4 receptor, the second specific binding partner and any HIV-1 in the test sample tropic for the CXCR4 receptor will bind and form a second specific binding partner-HIV-1 complex.

In an alternative embodiment of the present invention, the first mixture contains the test sample obtained from a subject that is being assessed for HIV-1 tropism and a single specific binding partner (for ease of reference, referred to as a "single specific binding partner") that comprises a chemokine receptor. The chemokine receptor is preferably the CXCR4 co-receptor protein. The order in which the test sample and the single specific binding partner are added to form the first mixture is not critical. The single specific binding partner and any HIV-1 in the test sample tropic for the CXCR4 receptor will bind to form a first specific binding panner-HIV-1 complex.

After the mixture containing the first specific binding partner-HIV-1 complex or the first specific binding partner-HIV-1 complex and the second specific binding partner-HIV-1 complex are formed, any unbound HIV-1 is removed from said complex or the complexes using any technique known in the art, such as washing.

Optionally, the single specific binding partner, the first specific binding partner, the second specific binding partner, the first specific binding partner-HIV-1 complex, the second specific binding partner-HIV-1 complex or any combinations thereof can be immobilized on a solid phase. The solid phase used in the assay (for the single specific binding partner, the first specific binding partner, the second specific binding partner, the first specific binding partner-HIV-1 complex, the second specific binding partner-HIV-1 complex or any combinations thereof) can be any solid phase known in the art, such as, but not limited to, a magnetic particle, a bead, a microparticle, a ligand, a receptor, a microtiter plate, a membrane, a scaffolding molecule, a disc or a chip.

Optionally, the single specific binding partner, first specific binding partner, the second specific binding partner, the first specific binding partner-HIV-1 complex, the second specific binding partner-HIV-1 complex or any combinations thereof can be labeled with one or more detectable labels. In terms of the detectable label, any detectable label known in the art can be used. For example, the detectable label can be a radioactive label (such as, e.g., ³H, ¹²⁵I, ³⁵S, ¹⁴C, ³²P, and ³³P), an enzymatic label (such as, e.g., horseradish peroxidase, alkaline peroxidase, glucose 6-phosphate dehydrogenase, and the like), a chemiluminescent label (such as, e.g., acridinium esters, luminal, isoluminol, thioesters, sulfonamides, phenanthridinium esters, and the like), a fluorescence label (such as, e.g., fluorescein (e.g., 5-fluorescein, 6-carboxyfluorescein, 3'6-carboxyfluorescein, 5(6)-carboxyfluorescein, 6-hexachloro-fluorescein, 6-tetrachlorofluorescein, fluorescein isothiocyanate, and the like)), rhodamine, phycobiliproteins, R-phycoerythrin, quantum dots (e.g., zinc sulfide-capped cadmium selenide), a thermometric label, or an immuno-polymerase chain reaction label. An introduction to labels, labeling procedures and detection of labels is found in Polak and Van Noorden, Introduction to Immunocytochemistry, 2nd ed., Springer Verlag, N.Y. (1997) and in Haugland, Handbook of Fluorescent Probes and Research Chemicals (1996) published by Molecular Probes, Inc., Eugene, Oregon. Methods for generating signals from detectable labels and measuring the resulting signal generated are well known to those skilled in the art. For example, a chemiluminescent signal can be generated after the addition of a basic solution. If more than one detectable label is used in the assays of the present invention, the detectable labels may be the same detectable labels or different detectable labels.

After any unbound HIV-1 is removed, the first specific binding partner-HIV-1 complex or the first specific binding partner-HIV-1 complex and the second specific binding partner-HIV-1 complex are separated from the mixture, using routine techniques known in the art such as by magnetic capture or by generating a signal from one or more detectable labels.

Once the first specific binding partner-HIV-1 complex or the first and second specific binding partner-HIV-1 complexes are separated from the mixture (and from one another if appropriate), the RNA from the respective complex or each of the complexes is isolated (e.g., from the first specific binding partner-HIV-1 complex or first specific binding partner-HIV-1 complex and the second specific binding partner-HIV-1 complex). The RNA in the complex or each of the complexes can be isolated using routine techniques known in the-art. For example, the RNA can be isolated by heating RNeasy extraction (e.g., such as by using a RNeasy kit (Qiagen Inc, Valencia, CA)) or by using an automated means such as, but not limited to, the m2000 sp system (Available from Abbott Molecular, Des Plaines, IL).

Once RNA from the requisite complex (e.g., first specific binding partner-HIV-1 complex) or each of the complexes (e.g., first specific binding partner-HIV-1 complex and second specific binding partner-HIV-1 complex) has been isolated, it can be quantified using routine techniques known in the art. Specifically, the RNA isolated from the requisite complex or each of the complexes can be quantified using Northern Blot Analysis, the techniques of which are well known to those skilled in the art.

Alternatively, the RNA isolated from the requisite complex or each of the complexes can be quantified using various types of polymerase chain reaction ("PCR"). One type of PCR that can be used is RT-PCR. RT-PCR is a technique that can be used to amplify, isolate, identify or quantify a known sequence from a cellular or tissue RNA. RT-PCR can be performed in either one or two steps. Generally, the technique involves synthesis of cDNA from RNA by reverse transcription ("RT") and then amplification of a specific cDNA by polymerase chain reaction ("PCR"). RT-PCR reactions employ the use of one of more primer sets (containing for example, a forward and a reverse primer) having specificity for the sequence of interest, a heat activated thermostable Taq polymerase, a reverse transcriptase, deoxyribonucleotide triphosphates ("dNTPs"), MgCl₂ and appropriate buffers, etc. all of which are well known to those skilled in the art.

Regardless of whether the RT-PCR is carried out in one or two steps, the RT step is run first and typically comprises a single temperature incubation at a temperature of between about 37°C and about 70°C. Different temperatures are appropriate for different RT enzymes and different primers, as is known to one skilled in the art. The subsequent PCR reaction typically comprises an initial incubation at about 94°C to about 96°C for about 4 to about 15 minutes. This step is used to denature the cDNA and also to activate the heat activated Taq polymerase. This is then followed by multiple cycles of amplification of the cDNA target. Three operations are performed during each cycle: target denaturation, primer annealing and primer extension. Target denaturation typically occurs at greater than about 90°C. Primer annealing temperature is dictated by the melting temperature of the specific primers used in the reaction and primer extension is performed at temperatures ranging from about 60°C to about 72°C depending on the thermostable Taq polymerase being used. When primer annealing and extension are performed at the same temperature, this is a two temperature PCR compared with a three temperature PCR in which each of the three steps occur at a different temperature. After the amplification phase is complete, a final extension time is typically added to ensure the synthesis of all amplification products.

Any method of detection can be used to detect the product of the RT or RT-PCR reaction. Methods for directly detecting the cDNA product of a RT reaction are well known to one skilled in the art and make use of detectable labels incorporated into or attached to the cDNA product. The detectable labels that can be used include those described previously herein as well as any other detectable labels known to those skilled in the art. A detectable label may be incorporated into the cDNA during its synthesis in the RT reaction, or it may be attached to the cDNA product after its synthesis. For example, the RT reaction can be carried out with labeled primers. One type of labeled primer has attached particles having a large number of signal generating molecules. Reverse transcription using a labeled nucleotide, such as dye-labeled UTP and/or CTP, incorporates a label into the transcribed nucleic acids. Alternatively, a post-synthesis coupling reaction can be used to detect the cDNA products. Attaching detectable labels to nucleic acids is well known to those of skill in the art and may be done by, for example, nick translation or end-labeling with, e.g. a labeled RNA or by treatment of the nucleic acid with kinase and subsequent attachment of a nucleic acid linker joining the sample nucleic acid to the label, e.g., a fluorophore. Methods for RT-PCR product detection include gel electrophoresis separation and ethidium bromide staining or detection of an incorporated detectable label in the product.

Methods that do not require a separation step prior to detection of the amplified product may also be used in the assays of the present invention, such as real time RT-PCR. Real time RT-PCR refers to the simultaneous amplification and quantification of specific RNA transcripts in a test sample. Many real time RT-PCR methods detect amplified product formation by monitoring changes in fluorescence by use of a detector probe during the amplification reaction. Examples of detector probes that can be used during real time RT-PCR include, but are not limited to, the 5'-exonuclease assay (TaqMan® probes (*See.* U.S. Patent No. 5,538,848)) various stern-loop molecular beacons (*See,* U.S. Patent Nos. 6,103,476 and 5,925,517 and Tyagi S., et al., Nature Biotechnology, 14:303-308 (1996)), stemless or linear beacons (*See*. WO 99/21881), PNA Molecular Beacons™ *(See.* U.S. Patent Nos. 6,355,421 and 6,593,091), linear PNA beacons (*See*, Kubista et al., SPIE, 4264:53-58 (2001)), non-FRET probes (*See.* U.S. Patent No. 6,150,097), Sunrise®/Amplifluor® probes (*See*, U.S. Patent No. 6,548,250), stem-loop and duplex Scorpion™ probes, etc. Detector probes can also comprise quenchers, including, but not limited to, black hole quenchers (Biosearch Technologies, Novato, CA), Iowa Black (Integrated DNA Technologies (IDT), Coralville, IA), QSY quencher (Molecular Probes, Eugene OR), and Dabsyl and Dabcel sulfonate/carboxylate Quenchers (Epoch Biosciences, Bothell, WA). In addition, intercalating labels can also be used such as ethidium bromide, SYBR® Green I (Molecular Probes, Eugene, OR), and PicoGreen® (Molecular Probes, Eugene, OR).

In addition to RT-PCR and real time RT-PCR, competitive RT-PCR and differential display RT-PCR can also be used in the assays of the present invention. Competitive RT-PCR involves adding a known amount of PCR-amplifiable standard into an RNA sample and then amplifying the standard and target RNAs in the same reaction. The exogenous standard and the endogenous target use the same primers for amplification, thus a competition for amplification components including primers, dNTPs, and polymerase ensues. If the exogenous standard is designed to be amplified at the same rate as the target sequence, then the ratio of products obtained from the endogenous and exogenous targets at the end of the amplification reflects the initial ratio of target to standard. Since the amount of exogenous standard added to the RT-PCR is known, the amount of endogenous target in the RNA sample can be determined (multiply the concentration of the input standard by the ratio of target-specific PCR product to standard-specific PCR product). Competitive RT-PCR is described in Gilliland, G. et al., PNAS, 87:2725-2729 (1990) and Becker-Andre, M., et al., Nucleic Acids. Res., 17:9437-9446 (1989)). Differential display RT-PCR ("DDRT-PCR") is a technique that allows for detecting differential expressed genes and is described in Liang P., et al., Science, 257:967-971 (1993); Bauer D., et al., Nucleic Acids Research, 21(18):4272-4280 (1993); U.S. Patent No. 5,599,672 and WO 94/01582.

Alternatively, the amount of RNA in the complex or each of the complexes can be quantitated using nucleic acid sequence based amplification ("NASBA"), which is described in EP 0329822 and U.S. Patent No. 5,409,818. More specifically, NASBA involves the isothermal amplification of the nucleic acid that involves the coordinated activities of three enzymes, AMV reverse transcriptase, RNaseH, and T7RNA polymerase. Quantitative detection is achieved by way of internal calibrators, which are added at isolation, co-amplified and subsequently identified along with the wild-type of RNA using a suitable detectable label.

Alternatively, the amount of RNA in the complex or each of the complexes can be quantitated using transcription mediated amplification ("TMA"). TMA employs an RNA polymerase to produce multiple RNA transcripts of a target region (*See*, U.S. Patent Nos. 55,480,784 and 5,399,491). Specifically, TMA uses a "promoter-primer" that hybridizes to a target nucleic acid in the presence of a reverse transcriptase and an RNA polymerase to form a double-stranded promoter from which the RNA polymerase produces RNA transcripts. These transcripts can become templates for further rounds of TMA in the presence of a second primer capable of hybridizing to the RNA transcripts. TMA is an isothermal method that uses an RNase H activity to digest the RNA strand of an RNA:DNA hybrid, thereby making the DNA strand available for hybridization with a primer or promoter-primer. Generally, the RNase H activity associated with the reverse transcriptase provided for amplification is used.

In one aspect, once the amount of RNA from the requisite complex or each of the complexes is quantitated (e.g., from the first specific binding partner-HIV-1 complex or the first specific binding partner-HIV-1 complex and the amount of RNA from second specific binding partner-HIV-1 complex), an appropriate ratio can then be determined or calculated (hereinafter "Calculated Ratio"). An example of a Calculated Ratio that can be used is the ratio of HIV-1 virus binding to the CCR5 co-receptor protein to the HIV-1 virus binding to the CXCR4 co-receptor protein. A further example of a Calculated Ratio that can be used is the ratio of HIV-1 RNA virus binding to a CXCR4 co-receptor protein to total HIV-1 RNA.

Once a Calculated Ratio is determined, the Calculated Ratio can then be compared with a predetermined ratio. For example, when the assay employs a first specific binding partner comprising a CCR5 co-receptor protein and a second specific binding partner comprising a CXCR4 co-receptor protein, an example of a predetermined ratio that can be used is the ratio of HIV-1 virus binding to a CCR5 co-receptor protein to HIV-1 virus binding to a CXCR4 co-receptor protein. A difference or change between a Calculated Ratio and this predetermined ratio (e.g., either positive or negative) would indicate a change in tropism in the HIV-1 population in the subject. For example, this change in tropism might be a change in the HIV-1 virus population binding from the CCR5 co-receptor protein to the CXCR4 co-receptor protein in the subject.

Alternatively, when the assay employs a specific binding partner comprising a CXCR4 co-receptor protein, an example of a predetermined ratio that can be used is the amount of HIV-1 virus binding to a CXCR4 co-receptor protein to total HIV-1 RNA. An increase in the Calculated Ratio compared to the predetermined ratio would indicate that the HIV-1 population has become tropic for the CXCR4 co-receptor protein.

In another aspect, once the amount of RNA from the requisite complex (e.g., from the first specific binding partner-HIV-1 complex) or each of the complexes (e.g., the first specific binding partner-HIV-1 complex and the second specific binding partner-HIV-1 complex) is quantitated, this amount or amounts can be compared with a predetermined level. For example, when the assay employs a specific binding partner comprising a CXCR4 co-receptor protein, an example of a predetermined level that can be used is the amount of RNA binding to a CXCR4 co-receptor protein. If the isolated RNA quantified from the specific binding partner-HIV-1 complex is less than the predetermined level, then the HIV-1 population is determined not to have become tropic for the CXCR4 co-receptor protein. However, if the isolated RNA quantified from the specific binding partner-HIV-1 complex is greater than the predetermined level, then the HIV-1 population is determined to have become tropic for the CXCR4 co-receptor protein.

As mentioned briefly previously herein, determining the tropism of an HIV-1 population in a test sample using the assays and kits of the present invention provides a clinician with useful information in determining the course of treatment in a subject as well as monitoring the course of treatment of a subject. For example, for a subject newly or recently diagnosed with HIV-1, the assays and kits of the present invention allow the clinician to determine whether or not the HIV-1 virus in the test sample obtained from the subject is tropic for the CCR5 or CXCR4 co-receptor and thus prescribe the appropriate course of treatment. Additionally, for a subject previously diagnosed with HIV-1 and who may be receiving treatment with one or more pharmaceutical compositions, the assays and kits of the present invention allow the clinician to monitor whether or not an HIV-1 population has become tropic for a different chemokine co-receptor or a variant of a different chemokine co-receptor. For example, a test sample can be obtained from a subject that is suspected of being infected with HIV-1. The assays of the present invention can be used to identify that the subject has been infected with an HIV-1 population tropic for the CCR5 co-receptor. The subject is then started on a treatment regimen comprising a CCR5 antagonist. During the course of treatment, the assays of the invention can be used to monitor whether or not the virus population in the subject may have become tropic for a different co-receptor or a variant of a different receptor, such as the CXCR4 co-receptor or variants of the CXCR4 co-receptor (*See,* for example. Westby M.. et al., J. of Virol., 80(10):4909-4920 (2006) who describe the emergence of CXCR4 variants in two patients initially harboring CCR5-tropic virus at baseline and who had received treatment for ten (10) days with maraviroc). Identifying such a change in tropism as early as possible during the course of treatment is preferable before the subject exhibits (a) an increase in HIV-1 viral load; (b) a decrease in CD4⁺ T count; or (c) a combination of both. Identification of change in tropism of an HI V-1 population in such a subject would allow the clinician to alter the subject's treatment regimen (e.g., stop the treatment regimen altogether or change or supplement the subject's current treatment to one or more different pharmaceutical compositions (e.g., a CXCR4 antagonist)).

### C. Assay Kits for Determining Tropism of HIV-1 for a Chemokine Receptor in a Test Sample

In another embodiment, the present invention relates to a test kit for determining the tropism of an HIV-1 population in a test sample. The kit contains a first specific binding partner, wherein said first specific binding partner comprises either a CCR5 co-receptor or a CXCR4 co-receptor. The test also contains a second specific binding partner, wherein said second specific binding partner comprises either a CCR5 co-receptor or a CXCR4 co-receptor, provided that the second specific binding partner is not identical to the first specific binding partner (e.g., the first specific binding partner comprises a CCR5 co-receptor and the second specific binding partner comprises a CXCR4 co-receptor or the first specific binding partner comprises a CXCR4 co-receptor and the second specific binding partner comprises a CCR5 co-receptor). The kit also contains one or more amplification reagents in order to allow the user to perform one or more amplification reactions in order to the quantify the amount ofRNA obtained from the test sample using any one of various types of PCR (such as, but not limited to, RT-PCR, competitive RT-PCR, real time RT-PCR or differential display RT-PCR), TMA or NASBA. The kit contains one or more amplification reagents selected from the group consisting of one or more primer sets specific for a sequence of interest, a heat activated thermostable Taq polymerase, AMV reverse transcriptase, RNAaseH, T7RNA polymerase, RNA polymerase, a reverse transcriptase, deoxyribonucleotide triphoshates, MgCl₂, one or more detector probes, and one or more buffers, wherein the amplification reagents allow the user to perform one or more amplification reactions in order to quantify the amount of HIV RNA obtained from the test sample using any one of various types of transcripted mediated amplification (TMR), nucleic acid sequence based amplification (NASBA), polymerase chain reaction (PCR) selected from the group consisting of RT-PCR, competitive RT-PCR, real time RT-PCR, differential display RT-PCR.

Optionally, the kit can also contain at least one detectable label. The detectable label can be a separate component of the kit. Alternatively, the detectable label may be conjugated to the first or second specific binding partner and supplied in the kit in thais form.

The kit also contains at least one solid phase. For example, the solid phase can be a magnetic particle, a bead, a microparticle, a ligand, a receptor, a microtiter plate, a membrane, a scaffolding molecule, a disc or a chip.

The kit also contains one or more instructions for determining the tropism of HIV-1 in a test sample. The kit can also contain instructions for performing one or more amplification reactions (e.g., PCR, TMA or NASBA). Such instructions can be provided in printed form or on CD, DVD, or other format of recorded media.

In the kit described above, the amount of RNA from an HIV-1 virus binding to (a) and the amount of RNA from an HIV-1 virus binding to (b) are measured.

### D. Adaptations of the Methods of the Present Invention

The invention as described herein also can be adapted for use in a variety of automated and semi-automated systems, e.g., as described in U.S. Patent Nos. 5,089,424, 5,006,309, U.S. Patent No. 5,294,404, and as commercially marketed by Abbott Laboratories (Abbott Park, 1L) including but not limited to, Abbott's m1000 (Real-Time PCR), m2000 (Real-Time PCR), ARCHITECT®, AxSYM®, IMx® , PRISM®, EIA (bead), and Quantum^{™} II instruments as well as other platforms. Moreover, the invention may be optionally adaptable for the Abbott Laboratories commercial Point of Care (i-STAT™) electrochemical immunoassay system for performing sandwich immunoassays. Immunosensors, and their methods of manufacture and operation in single-use test devices are described, for example in, U.S. Patent No. 5,063,081, U.S. Patent No. 7,419,821

U.S. Patent Publication 2004/0018577, U.S. Patent No. 7,682,833, and U.S. Patent No. 7,723,099.

In particular, with regard to the adaptation of the present assays to the I-STAT® system, the following configuration is preferred. A microfabricated silicon chip is manufactured with a pair of gold amperometric working electrodes and a silver-silver chloride reference electrode. On one of the working electrodes, polystyrene beads (0.2 mm diameter) with immobilized capture antibody are adhered to a polymer coating of patterned polyvinyl alcohol over the electrode. This chip is assembled into an I-STAT® cartridge with a fluidics format suitable for immunoassay. On a portion of the wall of the sample holding chamber of the cartridge there is a layer comprising the second detection antibody labeled with alkaline phosphatase (or other label). Within the fluid pouch of the cartridge is an aqueous reagent that includes p-aminophenol phosphate.

In operation, a sample suspected of containing HIV-1 is added to the holding chamber of the test cartridge and the cartridge is jnserted into the I-STAT® reader. After the second antibody (detection antibody) has dissolved into the sample, a pump element within the cartridge forces the sample into a conduit containing the chip. Here it is oscillated to promote formation of the sandwich between the first capture antibody. HIV-1, and the labeled second detection antibody. In the penultimate step of the assay, fluid is forced out of the pouch and into the conduit to wash the sample off the chip and into a waste chamber. In the final step of the assay, the alkaline phosphatase label reacts with p-aminophenol phosphate to cleave the phosphate group and permit the liberated p-aminophenol to be electrochemically oxidized at the working electrode. Based on the measured current, the reader is able to calculate the amount of analyte HIV-1 in the sample by means of an embedded algorithm and factory-determined calibration curve.

It goes without saying that the methods and kits described herein necessarily encompass other reagents and methods for carrying out the immunoassay. For instance, encompassed are various buffers such as are known in the art and/or which can be readily prepared or optimized to be employed, e.g., for washing, as a conjugate diluent, and/or as a calibrator diluent. An exemplary conjugate diluent is ARCHITECT® Human HIV-1 conjugate diluent (Abbott Laboratories, Abbott Park, IL) containing 2-(N-morpholino)ethanesulfonic acid (MES), other salt, protein blockers, antimicrobial and detergent. An exemplary calibrator diluent is ARCHITECT® Human HIV-1 calibrator diluent (Abbott Laboratories, Abbott Park, IL), which comprises a buffer containing MES, other salt, a protein blocker and an antimicrobial.

Furthermore, as previously mentioned, the methods and kits optionally are adapted for use on an automated or semi-automated system. Some of the differences between an automated or semi-automated system as compared to a non-automated system (e.g., ELISA) include the substrate to which the capture antibody is attached (which can impact sandwich formation and analyte reactivity), and the length and timing of the capture, detection and/or any optional wash steps. Whereas a non-automated format such as an ELISA may include a relatively longer incubation time with sample and capture reagent (e.g., about 2 hours) an automated or semi-automated format (e.g., ARCHITECT®) may have a relatively shorter incubation time (e.g., approximately 18 minutes for ARCHITECT®). Similarly, whereas a non-automated format such as an ELISA may incubate a detection antibody such as the conjugate reagent (Pb264) for a relatively longer incubation time (e.g., about 2 hours), an automated or semi-automated format (e.g., ARCHITECT®) may have a relatively shorter incubation time (e.g., approximately 4 minutes for the ARCHITECT®).

## Claims

1. An assay for detecting changes in tropism of an HIV-1 population in a test sample obtained from a subject, the assay comprising the steps of:
(a) contacting the test sample obtained from the subject with a first specific binding partner and a second specific binding partner to form a first specific binding partner-HIV-1 complex and a second specific binding partner-HIV-1 complex, wherein the first specific binding partner comprises a CCR5 co-receptor protein and the second specific binding partner comprises a CXCR4 co-receptor protein;
(b) separating the first specific binding partner-HIV-1 complex and second specific binding partner HIV-1 complex from the test sample;
(c) isolating RNA from the first specific binding partner-HIV-1 complex and RNA from the second specific binding partner-HIV-1 complex;
(d) quantifying the isolated RNA from the first specific binding partner-HIV-1 complex and the isolated RNA from the second specific binding partner-HIV-1 complex and determining a ratio of HIV-1 virus binding to CCR5 co-receptor protein to HIV-1 virus binding to CXCR4 co-receptor protein; and
(e) comparing the ratio determined in step (d) with a predetermined ratio of HIV-1 virus binding to a CCR5 co-receptor protein to HIV-1 virus binding to a CXCR4 co-receptor protein, wherein a change in the ratio compared to the predetermined ratio indicates a change in tropism in the HIV-1 population in the subject

2. The assay of claim 1, wherein the isolated RNA from the first specific binding partner-HIV-1 complex and the isolated RNA from the second specific binding partner-HIV-1 complex are quantified using PCR, nucleic acid sequence based amplification, tissue microarrays, transcription mediated amplification, nucleic acid sequence based amplification or Northern Blot analysis.

3. An assay for monitoring whether an HIV-1 population, which has infected a subject, become tropic for a CXCR4 co-receptor, the assay comprising the steps of:
(a) contacting a test sample obtained from the subject with a first specific binding partner and a second specific binding partner to form a first specific binding partner-HIV complex and a second specific binding partner-HIV-1 complex, wherein the first specific binding partner comprises a CCR5 co-receptor protein and the second specific binding partner comprises a CXCR4 co-receptor protein, further wherein said subject was previously identified as HIV-1 tropic for the CCR5 co-receptor;
(b) separating the first specific binding partner-HIV-1 complex and second specific binding partner HIV-1 complex from the test sample;
(c) isolating RNA from the first specific binding partner-HIV-1 complex and RNA from the second specific binding portner-HIV-1 complex;
(d) quantifying the isolated RNA from the first specific binding partner-HIV-1 complex and the isolated RNA from the second specific binding partner-HIV-1 complex, and determining a ratio of HIV-1 virus binding to CCR5 co-reccptor protein to HIV-1 virus binding to CXCR4 co-receptor protein; and
(e) comparing the ratio determined in step (d) with a predetermined ratio of HIV-1 virus binding to a CCR5 co-receptor protein to HIV-1 virus binding to a CXCR4 co-receptor protein, wherein an increase in the ratio of CXCR4 co-receptor protein or CXCR4 analog thereof compared to the predetermined ratio indicates a change in tropism of the HIV-1 population from the CCR5 co-receptor protein to the CXCR4 co-receptor protein in the subject.

4. The assay of claim 3, wherein the isolated RNA from the first specific binding partner-HIV-1 complex and the second specific binding partner-HIV-1 complex is quantified using PCR, nucleic acid sequence based amplification, tissue microarrays, transcription mediated amplification or Northern Blot analysis.

5. An assay for monitoring whether an HIV-1 population has become tropic for a CXCR4 co-receptor, the method comprising the steps of:
(a) contacting a test sample obtained from a subject with a specific binding partner to form a first specific binding partner-HIV-1 complex, wherein the specific binding partner comprises a CXCR4 co-receptor protein, and further wherein the specific binding partner is immobilized on a solid phase;
(b) separating the specific binding partner-HIV-1 complex from the test sample;
(c) isolating RNAfrom the specific binding partner-HIV-1 complex;
(d) quantifying the isolated RNA from the specific binding partner-HIV-1 complex and total HIV-1 RNA in the test sample and determining a ratio of RNA virus binding CXCR4 co-receptor protein to total HIV-1 RNA;
(e) comparing the ratio determined in step (d) with a predetermined ratio of HIV-1 virus binding to a CXCR4 co-receptor protein to total HIV-1 RNA, wherein an increase in the ratio of step (d) compared to the predetermined ratio indicates that the HIV-1 population has become tropic for the CXCR4 co-receptor protein.

6. An assay for monitoring whether an HIV-1 population has become tropic for a CXCR4 co-receptor, the method comprising the steps of:
(a) contacting a test sample obtained from a subject with a specific binding partner to form a first specific binding partner-HIV complex, wherein the specific binding partner comprises a CXCR4 co-receptor protein, and further wherein the specific binding partner is immobilized on a solid phase:
(b) separating the specific binding partner-HIV-1 complex from the test sample;
(c) isolating RNA from the specific binding partner-HIV-1 complex;
(d) quantifying the isolated RNA from the specific binding partner-HIV-1 complex; and
(e) comparing the quantity of RNA from the specific binding partner-HIV-1 complex with a predetermined level of RNA binding to CXCR4 co-receptor protein;
wherein if the isolated RNA quantified from the specific binding partner-HIV-1 complex is less than the predetermined level, then the HIV-1 population has not become tropic for the CXCR4 co-receptor protein, and further wherein if the isolated RNA quantified from the specific binding partner-HIV-1 complex is greater than the predetermined level, then the HIV-1 population has become tropic for the CXCR4 co-receptor protein.

7. The assay of claims 5 or 6, wherein the solid phase is selected from the group consisting of a magnetic particle, a non-magnetic particle, a membrane, a ligand, a receptor, a microparticle and a bead.

8. The assay of claims 5 or 6, wherein the isolated RNA from the specific binding partner-HIV-1 complex is quantified using PCR, nucleic acid sequence based amplification, tissue microarrays, transcription mediated amplification or Northern Blot analysis.

9. The assay of claims 2, 4 or 8, wherein the PCR is selected from the group of reverse transcriptase PCR, competitive RT-PCR, real time RT-PCR or differential display RT-PCR.

10. The assay of claims 3, 5 or 6, wherein the subject has been receiving treatment tor the HIV-1 with one or more pharmaceutical compositions.

11. The assay of claim 10, wherein the one or more pharmaceutical compositions is a CCR5 antagonist.

12. A kit for determining tropism of an HIV-1 population in a test sample obtained from a subject, the kit comprising:
(a) a first specific binding partner comprising a CXCR4 co-receptor;
(b) a second specific binding partner comprising a CCR5 co-receptor;
(c) at least one solid phase comprising (a) or (b);
(d) amplification reagents selected from the group consisting of one or more primer sets specific for a sequence of interest, a heat activated thermostable Taq polymerase, AMV reverse transcriptase, RNAaseH, T7RNA polymerase, RNA polymerase, a reverse transcriptase, deoxyribonucleotide triphoshates, MgCl₂, one or more detector probes, and one or more buffers, wherein the amplification reagents allow the user to perform one or more amplification reactions in order to quantify the amount of HIV RNA obtained from the test sample using any one of various types of transcripted mediated amplification (TMR), nucleic acid sequence based amplification (NASBA), polymerase chain reaction (PCR) selected from the group consisting of RT-PCR, competitive RT-PCR, real time RT-PCR, differential display RT-PCR; and
(e) instructions for using said kit,
wherein the amount of RNA from an HIV-1 virus binding to (a) and the amount of RNA from an HIV-1 virus binding to (b) are measured.

## Patentansprüche

1. Ein Assay zur Detektion von Änderungen im Tropismus einer HIV-1-Population in einer Testprobe, die von einem Subjekt erhalten wurde, wobei der Assay die folgenden Schritte umfasst:
(a) In-Kontakt-bringen der Testprobe, die von dem Subjekt erhalten wurde, mit einem ersten spezifischen Bindungspartner und einem zweiten spezifischen Bindungspartner, um einen ersten spezifischer-Bindungspartner-HIV-1-Komplex und einen zweiten spezifischer-Bindungspartner-HIV-1-Komplex zu bilden, worin der erste spezifische Bindungspartner ein CCR5-Co-Rezeptor-Protein umfasst und der zweite spezifische Bindungspartner ein CXCR4-Co-Rezeptor-Protein umfasst;
(b) Abtrennen des ersten spezifischer-Bindungspartner-HIV-1-Komplexes und des zweiten spezifischer-Bindungspartner-HIV-1-Komplexes aus der Testprobe;
(c) Isolieren der RNA aus dem ersten spezifischer-Bindungspartner-HIV-1-Komplex und der RNA aus dem zweiten spezifischer-Bindungspartner-HIV-1-Komplex;
(d) Quantifizieren der isolierten RNA aus dem ersten spezifischer-Bindungspartner-HIV-1-Komplex und der isolierten RNA aus dem zweiten spezifischer-Bindungspartner-HIV-1-Komplex und Bestimmen eines Verhältnisses von HIV-1-Virus, welches an CCR5-Co-Rezeptor-Protein bindet, zu HIV-1-Virus, welches an CXCR4-Co-Rezeptor-Protein bindet; und
(e) Vergleichen des Verhältnisses, das in Schritt (d) bestimmt wurde, mit einem vorher festgelegten Verhältnis von HIV-1-Virus, welches an CCR-5-Co-Rezeptor-Protein bindet, zu HIV-1-Virus, welches an CXCR4-Co-Rezeptor-Protein bindet, worin eine Änderung in dem Verhältnis verglichen mit dem vorher festgelegten Verhältnis eine Veränderung im Tropismus in der HIV-1-Population in dem Subjekt anzeigt.

2. Der Assay gemäß Anspruch 1, worin die isolierte RNA aus dem ersten spezifischer-Bindungspartner-HIV-1-Komplex und die isolierte RNA aus dem zweiten spezifischer-Bindungspartner-HIV-1-Komplex quantifiziert werden unter Verwendung von PCR, Nukleinsäuresequenz-basierter Amplifikation, Gewebe-Mikroarrays, Transkriptions-vermittelter Amplifikation, Nukleinsäuresequenz-basierter Amplifikation oder Northern Blot Analyse.

3. Ein Assay zur Überwachung ob eine HIV-1-Population, welche ein Subjekt infiziert hat, tropistisch wird für einen CXCR4-Co-Rezeptor, wobei der Assay die folgenden Schritte umfasst:
(a) In-Kontakt-bringen einer Testprobe, die von dem Subjekt erhalten wurde, mit einem ersten spezifischen Bindungspartner und einem zweiten spezifischen Bindungspartner, um einen ersten spezifischer-Bindungspartner-HIV-1-Komplex und einen zweiten spezifischer-Bindungspartner-HIV-1-Komplex zu bilden, worin der erste spezifische Bindungspartner ein CCRS-Co-Rezeptor-Protein umfasst, und der zweite spezifische Bindungspartner ein CXCR4-Co-Rezeptor-Protein umfasst, wobei weiterhin das Subjekt zuvor als HIV-1 tropistisch für den CCR5-Co-Rezeptor identifiziert wurde;
(b) Abtrennen des ersten spezifischer-Bindungspartner-HIV-1-Komplexes und des zweiten spezifischer-Bindungspartner-HIV-1-Komplexes aus der Testprobe;
(c) Isolieren der RNA aus dem ersten spezifischer-Bindungspartner-HIV-1-Komplex und der RNA aus dem zweiten spezifischer-Bindungspartner-HIV-1-Komplex;
(d) Quantifizieren der isolierten RNA aus dem ersten spezifischer-Bindungspartner-HIV-1-Komplex und der isolierten RNA aus dem zweiten spezifischer-Bindungspartner-HIV-1-Komplex und Bestimmen eines Verhältnisses von HIV-1-Virus, welches an CCR5-Co-Rezeptor-Protein bindet, zu HIV-1-Virus, welches an CXCR4-Co-Rezeptor-Protein bindet; und
(e) Vergleichen des Verhältnisses, das in Schritt (d) bestimmt wurde, mit einem vorher festgelegten Verhältnis von HIV-1-Virus, welches an CCR-5-Co-Rezeptor-Protein bindet, zu HIV-1-Virus, welches an CXCR4-Co-Rezeptor-Protein bindet, worin ein Anstieg in dem Verhältnis von CXCR4-Co-Rezeptor-Protein oder einem CXCR4-Analog davon verglichen mit dem vorher festgelegten Verhältnis eine Änderung im Tropismus der HIV-1-Population von dem CCR5-Co-Rezeptor-Protein zu dem CXCR4-Co-Rezeptor-Protein in dem Subjekt anzeigt.

4. Der Assay gemäß Anspruch 3, worin die isolierte RNA aus dem ersten spezifischer-Bindungspartner-HIV-1-Komplex und dem zweiten spezifischer-Bindungspartner-HIV-1-Komplex quantifiziert wird unter Verwendung von PCR, Nukleinsäuresequenz-basierter Amplifikation, Gewebe-Mikroarrays, Transkriptions-vermittelter Amplifikation oder Northern Blot Analyse.

5. Ein Assay zur Überwachung, ob eine HIV-1-Population tropistisch geworden ist für einen CXCR4-Co-Rezeptor, wobei das Verfahren folgende Schritte umfasst:
(a) In-Kontakt-bringen einer Testprobe, die von einem Subjekt erhalten wurde, mit einem spezifischen Bindungspartner, um einen ersten spezifischer-Bindungspartner-HIV-1-Komplex zu bilden, worin der spezifische Bindungspartner ein CXCR4-Co-Rezeptor-Protein umfasst, und weiter worin der spezifische Bindungspartner auf einer festen Phase immobilisiert ist;
(b) Abtrennen des spezifischer-Bindungspartner-HIV-1-Komplexes aus der Testprobe;
(c) Isolieren der RNA aus dem spezifischer-Bindungspartner-HIV-1-Komplex;
(d) Quantifizieren der isolierten RNA aus dem spezifischer-Bindungspartner-HIV-1-Komplex und der gesamt-HIV-1-RNA in der Testprobe, und Bestimmen eines Verhältnisses von RNA-Virus, welches CXCR4-Co-Rezeptor-Protein bindet, zu gesamt-HIV-1-RNA;
(e) Vergleichen des Verhältnisses, das in Schritt (d) bestimmt wurde, mit einem vorher festgelegten Verhältnis von HIV-1-Virus, welches an CXCR4-Co-Rezeptor-Protein bindet, zu gesamt-HIV-1-RNA, worin ein Anstieg in dem Verhältnis von Schritt (d) verglichen mit dem vorher festgelegten Verhältnis anzeigt, dass die HIV-1-Population tropistisch geworden ist für das CXCR4-Co-Rezeptor-Protein.

6. Ein Assay zur Überwachung ob eine HIV-1-Population tropistisch geworden ist für einen CXCR4-Co-Rezeptor, wobei das Verfahren die folgenden Schritte umfasst:
(a) In-Kontakt-bringen einer Testprobe, die von einem Subjekt erhalten wurde, mit einem spezifischen Bindungspartner, um einen ersten spezifischer-Bindungspartner-HIV-1-Komplex zu bilden, worin der spezifische Bindungspartner ein CXCR4-Co-Rezeptor-Protein umfasst, und weiter worin der spezifische Bindungspartner auf einer festen Phase immobilisiert ist;
(b) Abtrennen des spezifischer-Bindungspartner-HIV-1-Komplexes aus der Testprobe;
(c) Isolieren der RNA aus dem spezifischer-Bindungspartner-HIV-1-Komplex;
(d) Quantifizieren der isolierten RNA aus dem spezifischer-Bindungspartner-HIV-1-Komplex; und
(e) Vergleichen der Menge an RNA aus dem spezifischer-Bindungspartner-HIV-1-Komplex mit einem vorher festgelegten Level an RNA, welche an CXCR4-Co-Rezeptor-Protein bindet, worin, wenn die isolierte RNA, die aus dem spezifischer-Bindungspartner-HIV-1-Komplex quantifiziert wurde, geringer ist als der vorher festgelegte Level, dann die HIV-1-Population nicht tropistisch geworden ist für das CXCR4-Co-Rezeptor-Protein, und weiter, worin, wenn die isolierte RNA, die aus dem spezifischer-Bindungspartner-HIV-1-Komplex quantifiziert wurde, größer ist als der vorher festgelegte Level, dann die HIV-1-Population tropistisch geworden ist für das CXCR4-Co-Rezeptor-Protein.

7. Der Assay gemäß Ansprüchen 5 oder 6, worin die feste Phase gewählt ist aus der Gruppe bestehend aus einem magnetischen Partikel, einem nichtmagnetischen Partikel, einer Membran, einem Liganden, einem Rezeptor, einem Mikropartikel und einem Kügelchen.

8. Der Assay gemäß Ansprüchen 5 oder 6, worin die isolierte RNA aus dem spezifischer-Bindungspartner-HIV-1-Komplex quantifiziert wurde unter Verwendung von PCR, Nukleinsäuresequenz-basierter Amplifikation, Gewebe-Mikroarrays, Transkriptions-vermittelter Amplifikation oder Northern Blot Analyse.

9. Der Assay gemäß Ansprüchen 2, 4 oder 8, worin die PCR gewählt ist aus der Gruppe von reverse Transkriptase PCR, kompetitiver RT-PCR, Echtzeit-RT-PCR oder Differential Display-RT-PCR.

10. Der Assay gemäß Ansprüchen 3, 5 oder 6, worin das Subjekt eine Behandlung erhalten hat für das HIV-1 mit einer oder mehreren pharmazeutischen Zusammensetzungen.

11. Der Assay gemäß Anspruch 10, worin die eine oder die mehreren pharmazeutischen Zusammensetzungen ein CCR5-Antagonist sind.

12. Ein Kit zur Bestimmung von Tropismus einer HIV-1-Population in einer Testprobe, die von einem Subjekt erhalten wurde, wobei der Kit folgendes umfasst:
(a) einen ersten spezifischen Bindungspartner umfassend einen CXCR4-Co-Rezeptor;
(b) einen zweiten spezifischen Bindungspartner umfassend einen CCRS-Co-Rezeptor;
(c) mindestens eine feste Phase umfassend (a) oder (b);
(d) Amplifikationsreagenzien gewählt aus der Gruppe bestehend aus einem oder mehreren Primersets, die spezifisch sind für eine Sequenz von Interesse, eine Hitze-aktivierte thermostabilie Taq-Polymerase, AMV reverse Transkriptase, RNAaseH, T7RNA-Polymerase, RNA-Polymerase, einer reversen Transkriptase, Desoxyribonukleotidtriphosphaten, MgCl₂, einer oder mehreren Detektorsonden und einem oder mehreren Puffern, worin es die Amplifikationsreagenzien dem Verwender erlauben eine oder mehrere Amplifikationsreaktion durchzuführen, um die Menge an HIV-RNA zu quantifizieren, welche aus der Testprobe erhalten wird, unter Verwendung von irgendeiner von verschiedenen Typen von Transkriptions-vermittelter Amplifikation (TMR), Nukleinsäuresequenz-basierter Amplifikation (NASBA), Polymerase-Kettenreaktion (PCR) gewählt aus der Gruppe bestehend aus RT-PCR, kompetitiver RT-PCR, Echtzeit-RT-PCR, Differential Display-RT-PCR; und
(e) Anweisungen zur Verwendung des Kits,
worin die Menge an RNA von einem HIV-1-Virus, welches an (a) bindet und die Menge an RNA aus einem HIV-1-Virus, welches an (b) bindet, gemessen werden.

## Revendications

1. Test pour détecter des changements dans le tropisme d'une population de VIH-1 dans un échantillon d'essai obtenu à partir d'un sujet, le test comprenant les étapes de :
(a) mise en contact de l'échantillon d'essai obtenu à partir du sujet avec un premier partenaire de liaison spécifique et un second partenaire de liaison spécifique pour former un complexe premier partenaire de liaison spécifique-VIH-1 et un complexe second partenaire de liaison spécifique-VIH-1, où le premier partenaire de liaison spécifique comprend une protéine co-récepteur CCR5 et le second partenaire de liaison spécifique comprend une protéine co-récepteur CXCR4 ;
(b) séparation du complexe premier partenaire de liaison spécifique-VIH-1 et du complexe second partenaire de liaison spécifique VIH-1 de l'échantillon d'essai ;
(c) isolement d'ARN à partir du complexe premier partenaire de liaison spécifique-VIH-1 et d'ARN à partir du complexe second partenaire de liaison spécifique-VIH-1 ;
(d) quantification de l'ARN isolé à partir du complexe premier partenaire de liaison spécifique-VIH-1 et de l'ARN isolé à partir du complexe second partenaire de liaison spécifique-VIH-1 et détermination d'un rapport de virus VIH-1 se liant à la protéine co-récepteur CCR5 à un virus VIH-1 se liant à la protéine co-récepteur CXCR4 ; et
(e) comparaison du rapport déterminé dans l'étape (d) avec un rapport prédéterminé de virus VIH-1 se liant à une protéine co-récepteur CCR5 au virus VIH-1 se liant à une protéine co-récepteur CXCR4, où un changement dans le rapport comparé au rapport prédéterminé indique un changement dans le tropisme dans la population de VIH-1 chez le sujet.

2. Test selon la revendication 1, où l'ARN isolé à partir du complexe premier partenaire de liaison spécifique-VIH-1 et l'ARN isolé à partir du complexe second partenaire de liaison spécifique-VIH-1 sont quantifiés par PCR, amplification basée sur des séquences d'acide nucléique, micromatrices tissulaires, amplification à médiation par la transcription, amplification basée sur des séquences d'acide nucléique ou analyse par transfert de Northern.

3. Test pour examiner si une population de VIH-1, qui a infecté un sujet, devient tropique pour un co-récepteur CXCR4, le test comprenant les étapes de :
(a) mise en contact d'un échantillon d'essai obtenu à partir du sujet avec un premier partenaire de liaison spécifique et un second partenaire de liaison spécifique pour former un complexe premier partenaire de liaison spécifique-VIH et un complexe second partenaire de liaison spécifique-VIH-1, où le premier partenaire de liaison spécifique comprend une protéine co-récepteur CCR5 et le second partenaire de liaison spécifique comprend une protéine co-récepteur CXCR4, où, en outre, ledit sujet a été identifié préalablement comme tropique de VIH-1 pour le co-récepteur CCR5 ;
(b) séparation du complexe premier partenaire de liaison spécifique-VIH-1 et du complexe second partenaire de liaison spécifique VIH-1 de l'échantillon d'essai ;
(c) isolement d'ARN à partir du complexe premier partenaire de liaison spécifique-VIH-1 et d'ARN à partir du complexe second partenaire de liaison spécifique-VIH-1 ;
(d) quantification de l'ARN isolé à partir du complexe premier partenaire de liaison spécifique-VIH-1 et de l'ARN isolé à partir du complexe second partenaire de liaison spécifique-VIH-1, et détermination d'un rapport de virus VIH-1 se liant à la protéine co-récepteur CCR5 à un virus VIH-1 se liant à la protéine co-récepteur CXCR4 ; et
(e) comparaison du rapport déterminé dans l'étape (d) avec un rapport prédéterminé de virus VIH-1 se liant à une protéine co-récepteur CCR5 au virus VIH-1 se liant à une protéine co-récepteur CXCR4, où une augmentation dans le rapport de la protéine co-récepteur CXCR4 ou son analogue CXCR4 comparé au rapport prédéterminé indique un changement dans le tropisme de la population de VIH-1 de la protéine co-récepteur CCR5 à la protéine co-récepteur CXCR4 chez le sujet.

4. Test selon la revendication 3, où l'ARN isolé à partir du complexe premier partenaire de liaison spécifique-VIH-1 et l'ARN isolé à partir du complexe second partenaire de liaison spécifique-VIH-1 sont quantifiés par PCR, amplification basée sur des séquences d'acide nucléique, micromatrices tissulaires, amplification à médiation par la transcription ou analyse par transfert de Northern.

5. Test pour examiner si une population de VIH-1 est devenue tropique pour un co-récepteur CXCR4, le procédé comprenant les étapes de :
(a) mise en contact d'un échantillon d'essai obtenu à partir d'un sujet avec un partenaire de liaison spécifique pour former un premier complexe partenaire de liaison spécifique-VIH-1, où le partenaire de liaison spécifique comprend une protéine co-récepteur CXCR4 et où, en outre, le partenaire de liaison spécifique est immobilisé sur une phase solide ;
(b) séparation du complexe partenaire de liaison spécifique-VIH-1 de l'échantillon d'essai ;
(c) isolement d'ARN à partir du complexe partenaire de liaison spécifique-VIH-1 ;
(d) quantification de l'ARN isolé à partir du complexe partenaire de liaison spécifique-VIH-1 et de l'ARN de VIH-1 total dans l'échantillon d'essai et détermination d'un rapport de virus à ARN se liant à une protéine co-récepteur CXCR4 à l'ARN de VIH-1 total ;
(e) comparaison du rapport déterminé dans l'étape (d) avec un rapport prédéterminé de virus VIH-1 se liant à une protéine co-récepteur CXCR4 à l'ARN de VIH-1 total, où une augmentation dans le rapport de l'étape (d) comparé au rapport prédéterminé indique que la population de VIH-1 est devenue tropique pour la protéine co-récepteur CXCR4.

6. Test pour examiner si une population de VIH-1 est devenue tropique pour un co-récepteur CXCR4, le procédé comprenant les étapes de :
(a) mise en contact d'un échantillon d'essai obtenu à partir d'un sujet avec un partenaire de liaison spécifique pour former un premier complexe partenaire de liaison spécifique-VIH, où le partenaire de liaison spécifique comprend une protéine co-récepteur CXCR4 et où, en outre, le partenaire de liaison spécifique est immobilisé sur une phase solide ;
(b) séparation du complexe partenaire de liaison spécifique-VIH-1 de l'échantillon d'essai ;
(c) isolement d'ARN à partir du complexe partenaire de liaison spécifique-VIH-1 ;
(d) quantification de l'ARN isolé à partir du complexe partenaire de liaison spécifique-VIH-1 ; et
(e) comparaison de la quantité d'ARN provenant du complexe partenaire de liaison spécifique-VIH-1 avec un niveau prédéterminé d'ARN se liant à une protéine co-récepteur CXCR4 où, si l'ARN isolé quantifié à partir du complexe partenaire de liaison spécifique-VIH-1 est inférieur au niveau prédéterminé, alors la population de VIH-1 n'est pas devenue tropique pour la protéine co-récepteur CXCR4, et où, en outre, si l'ARN isolé quantifié à partir du complexe partenaire de liaison spécifique-VIH-1 est supérieur au niveau prédéterminé, alors la population de VIH-1 est devenue tropique pour la protéine co-récepteur CXCR4.

7. Test selon la revendication 5 ou 6, où la phase solide est choisie dans le groupe consistant en une particule magnétique, une particule non magnétique, une membrane, un ligand, un récepteur, une microparticule et une bille.

8. Test selon la revendication 5 ou 6, où l'ARN isolé à partir du complexe partenaire de liaison spécifique-VIH-1 est quantifié par PCR, amplification basée sur des séquences d'acide nucléique, micromatrices tissulaires, amplification à médiation par la transcription ou analyse par transfert de Northern.

9. Test selon la revendication 2, 4 ou 8, où la PCR est choisie dans le groupe de la PCR à transcriptase inverse, la RT-PCR compétitive, la RT-PCR en temps réel ou la RT-PCR à présentation différentielle.

10. Test selon la revendication 3, 5 ou 6, où le sujet a reçu un traitement pour le VIH-1 avec une ou plusieurs compositions pharmaceutiques.

11. Test selon la revendication 10 où la une ou plusieurs compositions pharmaceutiques est un antagoniste de CCR5.

12. Kit pour déterminer un tropisme d'une population de VIH-1 dans un échantillon d'essai obtenu à partir d'un sujet, le kit comprenant :
(a) un premier partenaire de liaison spécifique comprenant un co-récepteur CXCR4 ;
(b) un second partenaire de liaison spécifique comprenant un co-récepteur CCR5 ;
(c) au moins une phase solide comprenant (a) ou (b) ;
(d) des réactifs d'amplification choisis dans le groupe consistant en une ou plusieurs séries d'amorces spécifiques pour une séquence d'intérêt, une Taq polymérisable thermostable activée par la chaleur, une transcriptase inverse de AMV, une ARNaseH, une ARN polymérase de T7, une ARN polymérase, une transcriptase inverse, des désoxyribonucléotide triphosphates, MgCl₂, une ou plusieurs sondes détectrices, et un ou plusieurs tampons, où les réactifs d'amplification permettent à l'utilisateur de réaliser une ou plusieurs réactions d'amplification afin de quantifier la quantité d'ARN de VIH obtenue à partir de l'échantillon d'essai au moyen de l'un quelconque de différents types d'amplification à médiation par la transcription (TMR), d'amplification basée sur des séquences d'acide nucléique (NASBA), de réaction en chaîne par polymérase(PCR) choisie dans le groupe consistant en la RT-PCR, la RT-PCR compétitive, la RT-PCR en temps réel, la RT-PCR à présentation différentielle ; et
(e) des instructions pour utiliser ledit kit,
où la quantité d'ARN d'un virus VIH-1 se liant à (a) et la quantité d'ARN d'un virus VIH-1 se liant à (b) sont mesurées.
